# EUROPEAN PATENT APPLICATION

(11) **EP 4 831 206 A1**
(43) Date of publication of application: **28.10.2026**
(21) Application number: 25806641.4
(22) Date of filing: 28.03.2025
(51) Int. Cl.: C07K 16/28, C07K 14/74, A61K 35/17, A61P 35/00

(54) **HLA-G-TARGETING ANTIBODY OR ANTIGEN-BINDING FRAGMENT THEREOF AND USE THEREOF**

(30) Priority: 24.05.2024 CN 202410656768
(71) Applicant: Hefei TG Immunopharma Co., Ltd., Hefei, Anhui 230001 (CN)
(72) Inventor: LI, Yangyang, Hefei, Anhui 230001 (CN); CAO, Guoshuai, Hefei, Anhui 230001 (CN); CHENG, Ying, Hefei, Anhui 230001 (CN); WU, Yuwei, Hefei, Anhui 230001 (CN)
(74) Representative: Studio Torta S.p.A.
(86) International application number: PCT/CN2025/085669
(87) International publication number: WO 2025/241702

(57) **Abstract**

Provided are an HLA-G-targeting antibody or antigen-binding fragment thereof, and use thereof, relating to the field of antibodies. The antibody or antigen-binding fragment thereof includes a heavy chain complementarity determining region (HCDR) and a light chain complementarity determining region (LCDR). The HCDR includes at least one amino acid sequence selected from SEQ ID NO: 1 to SEQ ID NO: 6, or an amino acid sequence having at least 80% homology thereto; and the LCDR includes at least one amino acid sequence selected from SEQ ID NO: 7 to SEQ ID NO: 12, or an amino acid sequence having at least 80% homology thereto. The antibody or antigen-binding fragment thereof has high specificity and high affinity, is capable of binding to both human and cynomolgus monkey HLA-G, specifically blocks binding of HLA-G to its receptors LILRB1 and LILRB2, and promotes killing of tumor cells by immune cells, thereby effectively treating and/or preventing an HLA-G-mediated related disease, such as a tumor.

## Description

### FIELD

The present disclosure belongs to the field of antibodies, and in particular, relates to an antibody targeting HLA-G or antigen-binding fragment thereof and use thereof.

### BACKGROUND

In recent years, anti-tumor immunotherapies targeting immune checkpoints have achieved tremendous success in clinical applications. Several humanized or fully human monoclonal antibodies targeting CTLA-4 and PD-1/PD-L1, which are representative immune checkpoint molecules, have been approved for marketing and are used for the treatment of various tumors. Nevertheless, the overall response rate among patients remains only about 30%, leaving a large number of clinical patients without therapeutic benefit. Therefore, there remains a need to identify novel immune checkpoint molecules and develop new combination therapeutic strategies.

HLA-G is a non-classical major histocompatibility complex class I (MHC-I) molecule that is primarily expressed in placental tissues, while its expression in other normal tissues is either absent or extremely low. However, HLA-G is highly expressed in many tumor types, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma. Furthermore, HLA-G expression is negatively correlated with patient prognosis.

Studies have reported that HLA-G can bind to its receptors LILRB1 (ILT2) and LILRB2 (ILT4), thereby suppressing the effector functions of natural killer (NK) cells and T cells. By blocking the binding of HLA-G to LILRB1 or LILRB2 using a blocking antibody, the anti-tumor functions of NK cells and T cells can be significantly restored. Therefore, targeting the HLA-G signaling pathway holds great application prospects.

Currently, clinical research on HLA-G remains relatively limited, and there is an urgent need to develop an anti-HLA-G antibody with high affinity and favorable therapeutic efficacy.

### SUMMARY

The present disclosure is based on the inventors' findings of the following problems and facts.

HLA-G is a non-classical MHC-I molecule that is primarily expressed in placental tissues, while its expression in other normal tissues is either absent or extremely low. However, HLA-G is highly expressed in many tumor types, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma. Furthermore, HLA-G expression is negatively correlated with patient prognosis.

Studies have reported that HLA-G can bind to its receptors LILRB1 (ILT2) and LILRB2 (ILT4), thereby suppressing the effector functions of NK cells and T cells. By blocking the binding of HLA-G to LILRB1 or LILRB2 using a blocking antibody, the anti-tumor functions of NK cells and T cells can be significantly restored.

The inventors of the present disclosure have successfully obtained blocking antibodies that specifically bind to HLA-G through screening. Specifically, in this study, a high-affinity monoclonal antibody that specifically binds to HLA-G and blocks the binding of HLA-G to its receptors LILRB1 or LILRB2 was identified using hybridoma technology. The constant regions of the murine anti-HLA-G monoclonal antibody and the framework regions in the variable regions of the murine antibody were humanized, thereby obtaining a humanized anti-HLA-G antibody.

Experimental verification has shown that both the murine antibody and the humanized antibody obtained in the present disclosure can specifically target and bind to HLA-G, block the binding of HLA-G to its receptors LILRB1 or LILRB2, and promote immune cell-mediated killing of tumor cells, thereby effectively treating and/or preventing an HLA-G-mediated related disease, such as a tumor.

Specifically, the present disclosure provides the following technical solutions:
In a first aspect, the present disclosure provides an antibody or antigen-binding fragment thereof. According to an embodiment of the present disclosure, the antibody or antigen-binding fragment thereof includes a heavy chain complementarity determining region (HCDR) and a light chain complementarity determining region (LCDR). The HCDR includes at least one amino acid sequence selected from SEQ ID NO: 1 to SEQ ID NO: 6, or an amino acid sequence having at least 80% homology thereto; and the LCDR includes at least one amino acid sequence selected from SEQ ID NO: 7 to SEQ ID NO: 12, or an amino acid sequence having at least 80% homology thereto. In embodiments of the present disclosure, the aforementioned antibody or antigen-binding fragment exhibits high specificity and high affinity, is capable of binding to both human and cynomolgus monkey HLA-G, specifically blocks the binding of HLA-G to its receptors LILRB1 and LILRB2, and promotes immune cell-mediated killing of tumor cells, thereby effectively treating and/or preventing an HLA-G-mediated related disease, such as a tumor.
In a second aspect, the present disclosure provides a nucleic acid molecule. According to an embodiment of the present disclosure, the nucleic acid molecule encodes the antibody or antigen-binding fragment thereof according to the first aspect of the present disclosure. In embodiments of the present disclosure, *in vitro* large-scale preparation of the aforementioned antibody or antigen-binding fragment thereof can be achieved based on the aforementioned nucleic acid molecule.
In a third aspect, the present disclosure provides an expression vector. According to an embodiment of the present disclosure, the expression vector carries the nucleic acid molecule according to the second aspect of the present disclosure. In embodiments of the present disclosure, the expression vector can efficiently express the aforementioned antibody or antigen-binding fragment thereof in a suitable host cell.
In a fourth aspect, the present disclosure provides a method for preparing the antibody or antigen-binding fragment thereof according to the first aspect. According to an embodiment of the present disclosure, the method includes: introducing the expression vector according to the third aspect of the present disclosure into a cell; and culturing the cell under conditions suitable for protein expression and secretion, to obtain the antibody or antigen-binding fragment thereof. In embodiments of the present disclosure, the aforementioned antibody or antigen-binding fragment thereof can be efficiently prepared by the aforementioned method.
In a fifth aspect, the present disclosure provides a recombinant cell. According to an embodiment of the present disclosure, the recombinant cell carries the antibody or antigen-binding fragment thereof according to the first aspect, the nucleic acid molecule according to the second aspect, or the expression vector according to the third aspect of the present disclosure. The recombinant cell is obtained by transfecting or transforming the expression vector. In embodiments of the present disclosure, the recombinant cell can efficiently express the aforementioned antibody or antigen-binding fragment thereof under suitable conditions.
In a sixth aspect, the present disclosure provides an immunoconjugate. According to an embodiment of the present disclosure, the immunoconjugate includes: the antibody or antigen-binding fragment thereof according to the first aspect, the nucleic acid molecule according to the second aspect, the expression vector according to the third aspect, or the recombinant cell according to the fifth aspect of the present disclosure. As mentioned above, the antibody or antigen-binding fragment can effectively and specifically bind to both human and cynomolgus monkey HLA-G proteins. Therefore, the immunoconjugate including the antibody or antigen-binding fragment can likewise bind to human and cynomolgus monkey HLA-G proteins. The aforementioned immunoconjugate exhibits favorable efficacy in preventing and/or treating HLA-G-mediated tumor diseases, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma.
In a seventh aspect, the present disclosure provides a composition. According to an embodiment of the present disclosure, the composition includes: the antibody or antigen-binding fragment thereof according to the first aspect, the nucleic acid molecule according to the second aspect, the expression vector according to the third aspect, the recombinant cell according to the fifth aspect, or the immunoconjugate according to the sixth aspect of the present disclosure. As mentioned above, the antibody or antigen-binding fragment can effectively bind to both human and cynomolgus monkey HLA-G. Therefore, the composition including the antibody or antigen-binding fragment, such as a food composition or a pharmaceutical composition, can likewise effectively treat or prevent tumor diseases, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma.
In an eighth aspect, the present disclosure provides use of the antibody or antigen-binding fragment thereof according to the first aspect, the nucleic acid molecule according to the second aspect, the expression vector according to the third aspect, the recombinant cell according to the fifth aspect, the immunoconjugate according to the sixth aspect, or the composition according to the seventh aspect in the manufacture of a medicament for treating or preventing an HLA-G-mediated related disease. As mentioned above, the antibody or antigen-binding fragment can effectively bind to both human and cynomolgus monkey HLA-G. Therefore, the medicament including the aforementioned antibody or antigen-binding fragment and related substances likewise exhibit significant efficacy in treating and/or preventing HLA-G-mediated tumor diseases, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma.
In a ninth aspect, the present disclosure provides a medicament. According to an embodiment of the present disclosure, the medicament includes: the antibody or antigen-binding fragment thereof according to the first aspect, the nucleic acid molecule according to the second aspect, the expression vector according to the third aspect, the recombinant cell according to the fifth aspect, the immunoconjugate according to the sixth aspect, or the composition according to the seventh aspect. The medicament is for use in treating or preventing an HLA-G-mediated related disease. As mentioned above, the antibody or antigen-binding fragment can effectively bind to both human and cynomolgus monkey HLA-G. Therefore, the medicament including the aforementioned antibody or antigen-binding fragment and related substances likewise exhibit significant efficacy in treating and/or preventing the HLA-G-mediated tumor diseases, such as breast cancer, colorectal cancer, cervical cancer, endometrial cancer, esophageal cancer, gastric cancer, hepatocellular carcinoma, lung cancer, nasopharyngeal carcinoma, ovarian cancer, or renal cell carcinoma.
In a tenth aspect, the present disclosure provides use of the antibody or antigen-binding fragment thereof according to the first aspect in the manufacture of a kit for detecting HLA-G. As mentioned above, the antibody or antigen-binding fragment can effectively bind to both human and cynomolgus monkey HLA-G. Therefore, the kit including the aforementioned antibody or antigen-binding fragment can be used for effectively detecting HLA-G. In embodiments of the present disclosure, the aforementioned kit can be used for scientific research, such as qualitative or quantitative detection of HLA-G proteins in a biological sample.
In an eleventh aspect, the present disclosure provides a kit. According to an embodiment of the present disclosure, the kit includes the antibody or antigen-binding fragment thereof according to the first aspect. As mentioned above, the antibody or antigen-binding fragment can effectively bind to both human and cynomolgus monkey HLA-G. Therefore, the kit including the aforementioned antibody or antigen-binding fragment can be used for effectively detecting HLA-G. In embodiments of the present disclosure, the kit can be used for scientific research, such as qualitatively or quantitatively detecting HLA-G proteins in a biological sample, and can also be used for determining the status of an individual, for example, by determining the HLA-G level of the individual and evaluating whether the HLA-G level is higher or lower than a normal level.

Additional aspects and advantages of the present disclosure will be provided in part in the following description, or will become apparent in part from the following description, or can be learned from the practice of the present disclosure.

### BRIEF DESCRIPTION OF DRAWINGS

In order to more clearly illustrate the technical solutions according to the embodiments of the present disclosure, drawings used in the description of the embodiments are briefly described below. Obviously, the drawings described below merely illustrate some embodiments of the present disclosure. Based on these drawings, other drawings can be obtained by those skilled in the art without creative effort.
FIG. 1 shows enzyme-linked immunosorbent assay (ELISA) results demonstrating binding of the murine antibody to human and cynomolgus monkey HLA-G proteins according to an embodiment of the present disclosure.
FIG. 2 shows flow cytometry results demonstrating binding of the murine antibody to the JEG-3 tumor cell line with high HLA-G expression according to an embodiment of the present disclosure.
FIG. 3 shows flow cytometry results demonstrating blocking the binding of LILRB1 protein or LILRB2 protein to CHO-K1-HLA-G cells by the murine antibody according to an embodiment of the present disclosure.
FIG. 4 shows ELISA results demonstrating blocking the binding of HLA-G protein to LILRB1 protein or LILRB2 protein by the murine antibody according to an embodiment of the present disclosure.
FIG. 5 shows flow cytometry results demonstrating promoting the PBMC-mediated killing of SK-OV-3 cells overexpressing HLA-G by the murine antibody according to an embodiment of the present disclosure.
FIG. 6 shows flow cytometry results demonstrating binding of the humanized antibody to the JEG-3 tumor cell line with high HLA-G expression according to an embodiment of the present disclosure.
FIG. 7 shows flow cytometry results demonstrating blocking the binding of LILRB1 protein or LILRB2 protein to CHO-K1-HLA-G cells by the humanized antibody according to an embodiment of the present disclosure.
FIG. 8 shows ELISA results demonstrating blocking the binding of HLA-G protein to LILRB1 protein or LILRB2 protein by the humanized antibody according to an embodiment of the present disclosure.
FIG. 9 shows flow cytometry results demonstrating promoting the PBMC-mediated killing of K562 cells overexpressing HLA-G by the humanized antibody provided according to an embodiment of the present disclosure.

### DETAILED DESCRIPTION

Embodiments of the present disclosure are described in detail below, examples of which are illustrated in the accompanying drawings. The embodiments described below with reference to the drawings are exemplary and are intended to be illustrative of the present disclosure and are not to be construed as limiting the present disclosure.

As used herein, the terms "first" and "second" are used for descriptive purposes only and are not to be construed as indicating or implying relative importance, or implicitly indicating the number of technical features indicated. Thus, a feature associated with "first" and "second" may explicitly or implicitly includes at least one such feature. As used herein, the term "plurality", "more", or "multiple" means at least two, e.g. two, three, and so on, unless otherwise expressly specified.

As used herein, the term "antibody" refers to an immunoglobulin having a tetrapeptide chain structure composed of two identical heavy chains and two identical light chains linked via interchain disulfide bonds. The amino acid composition and arrangement of heavy chain constant regions of an immunoglobulin varies, resulting in differences in antigenicity. Immunoglobulins may be divided into five classes: IgM, IgD, IgG, IgA, and IgE. Immunoglobulins of the same class may further be divided into different subclasses according to their amino acid composition, such as IgG1, IgG2, IgG3, and IgG4. Immunoglobulin light chains are classified into κ chains or λ chains based on their different constant regions.

In the present disclosure, a heavy chain variable region of an antibody may further include a heavy chain constant region, and the heavy chain constant region includes human/murine IgG1, IgG2, IgG3, IgG4, or variants thereof.

In the present disclosure, a light chain variable region of an antibody may further include a light chain constant region, and the light chain constant region includes human/murine κ chain, λ chain, or variants thereof.

The sequence of approximately the first 110 amino acids near the N-terminus of antibody heavy and light chains varies greatly and constitutes a variable region (Fv region). The remaining amino acid sequences near the C-terminus are relatively conserved and constitute a constant region. The variable region includes three hypervariable regions (HVRs) and four relatively conserved framework regions (FRs). The three HVRs determine antibody specificity and are also known as complementarity determining regions (CDRs). Each light chain variable region (LCVR) and heavy chain variable region (HCVR) consists of three CDRs and four FRs, arranged from the amino terminus to the carboxyl terminus in the following order: FR1, CDR1, FR2, CDR2, FR3, CDR3, and FR4. The three CDRs of the light chain are designated LCDR1, LCDR2, and LCDR3. The three CDRs of the heavy chain are designated HCDR1, HCDR2, and HCDR3.

In the present disclosure, the antibody may be from a rabbit antibody, a murine antibody, a sheep antibody, a goat antibody, a primate (including human and monkey) antibody, or a mutant thereof, or the antibody is a chimeric antibody from antibodies of different origins.

Using the murine antibody as an example, the anti-HLA-G monoclonal antibody of the present disclosure is described below. A "murine antibody" refers to a monoclonal antibody against human HLA-G prepared according to the knowledge and skills known in the art. During preparation, an antigen is injected into a test subject, and then hybridomas expressing antibodies with desired sequences or functional characteristics are isolated. In a preferred embodiment of the present disclosure, a murine HLA-G antibody or antigen-binding fragment thereof may further include a light chain constant region of a murine κ chain, λ chain, or a variant thereof, or may further include a heavy chain constant region of murine IgG1, IgG2, IgG3, or a variant thereof.

As used herein, the term "chimeric antibody" refers to an antibody generated by fusing the variable region of a murine antibody with the constant region of a human antibody, thereby reducing immune responses induced by the murine antibody. Genes encoding the variable regions are cloned from mouse hybridoma cells, and then genes encoding the constant region of a human antibody are cloned as needed. Subsequently, the mouse variable region genes are linked to the human constant region genes to form chimeric genes, which are then inserted into an expression vector and finally expressed in a eukaryotic expression system or a prokaryotic expression system. In a preferred embodiment of the present disclosure, the light chain of the HLA-G chimeric antibody further includes the light chain Fc region of a human κ or λ chain, or a variant thereof. The heavy chain of the HLA-G chimeric antibody further includes the heavy chain constant region of human IgG1, IgG2, IgG3, IgG4, or a variant thereof.

As used herein, the term "antigen-binding fragment" refers to a Fab fragment, an Fab' fragment, an F(ab')2 fragment, an Fv fragment, or single-chain Fv (scFv) fragment having antigen-binding activity and binding to human HLA-G. The term "antigen-binding fragment" includes one or more CDRs selected from SEQ ID NO: 1 to SEQ ID NO: 12 of the antibody described in the present disclosure. The Fv fragment contains a heavy chain variable region and a light chain variable region of the antibody but lacks a constant region, and represents the smallest antibody fragment that retains the entire antigen-binding site. Generally, an Fv antibody further includes a polypeptide linker between VH and VL domains, and is capable of forming a structure required for antigen binding. Alternatively, two antibody variable regions can be linked by different linkers into a single polypeptide chain, which is referred to as a single-chain antibody or single-chain Fv.

In the present disclosure, the amino acid sequences of the listed CDRs are shown according to the Kabat numbering system. However, it is well known to those skilled in the art that CDRs of antibodies can be defined by various methods in the art, such as IMGT numbering system or Chothia numbering system. It should be understood by those skilled in the art that, unless otherwise specified, the terms "CDR" and "complementarity determining region" for a given antibody or region thereof (e.g., a variable region) should be understood to encompass complementarity determining regions defined by any of the known numbering system described above in the present disclosure. Although the scope of protection claimed in the present disclosure is based on the sequences shown according to the Kabat numbering system, amino acid sequences corresponding to other CDR numbering system should also be included within the protection scope of the present disclosure.

As used herein, the term "amino acid" is represented by a single-letter or three-letter code, having the following meanings: A: Ala (alanine); R: Arg (arginine); N: Asn (asparagine); D: Asp (aspartic acid); C: Cys (cysteine); Q: Gln (glutamine); E: Glu (glutamic acid); G: Gly (glycine); H: His (histidine); I: Ile (isoleucine); L: Leu (leucine); K: Lys (lysine); M: Met (methionine); F: Phe (phenylalanine); P: Pro (proline); S: Ser (serine); T: Thr (threonine); W: Trp (tryptophan); Y: Tyr (tyrosine); and V: Val (valine).

For nucleic acids, the terms "homology", "identity", or "similarity" are used to describe or compare the degree of nucleotide similarity between two or more nucleotide sequences. The percentage of "sequence homology" between a first sequence and a second sequence may be calculated by dividing the number of nucleotides in the first sequence that are identical to corresponding nucleotides in the second sequence by the total number of nucleotides in the first sequence and multiplying the result by 100%. The deletion, insertion, substitution, or addition of each nucleotide in the second sequence relative to the first sequence is considered a difference at a single nucleotide (position). Alternatively, the degree of sequence identity between two or more nucleotide sequences can be calculated, under standard settings, using known computer algorithms for sequence alignment, such as NCBI Blast v2.0. Additional techniques, computer algorithms, and settings for determining the degree of sequence identity are described, for example, in WO 04/037999, EP 0 967 284, EP 1 085 089, WO 00/55318, WO 00/78972, WO 98/49185 and GB 2357768-A.

For polypeptides, the terms "(substantial) homology", "identity", or "similarity" are used to describe or compare the degree of amino acid similarity between two or more polypeptides or designated sequences when optimally aligned and compared (with appropriate insertions or deletions of nucleotides). The percentage homology between two sequences varies with the number of identical positions shared by the sequences upon optimal alignment (i.e., % homology = number of identical positions/total number of positions × 100), where optimal alignment is determined by taking into account the number and length of gaps required to achieve the optimal alignment. Sequence comparison and percent identity determination between two sequences can be accomplished using mathematical algorithms, as described in the non-limiting examples herein.

In the present disclosure, without substantially affecting antibody activity (retaining at least 95% of activity), those skilled in the art can substitute, add, and/or delete one or more (e.g., 1, 2, 3, 4, 5, 6, 7, 8, 9, 10, or more) amino acids in the amino acid sequence of the present disclosure, to obtain variants of the sequence of the antibody or functional fragments thereof. Such variants are considered to be included within the scope of the present disclosure. For example, amino acids having similar properties in the variable region may be substituted. The variant sequence described in the present disclosure may have at least 80% identity (or homology) to a reference sequence, meaning at least 80% identity to each reference sequence, for example, 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% identity. The sequence identity described in the present disclosure may be measured using sequence analysis software, for example, the BLAST computer program, particularly BLASTP or TBLASTN, using default parameters. The amino acid sequences mentioned in the present disclosure are all shown from the N-terminus to the C-terminus.

As used herein, the term "specifically binding" refers to a non-random binding interaction between two molecules, such as the interaction between an antibody and its target antigen. In some embodiments, an antibody that specifically binds to an antigen (or an antibody specific for an antigen) refers to an antibody that binds to the antigen with an affinity (KD) of less than about 10⁻⁵ M, for example, less than about 10⁻⁶ M, 10⁻⁷ M, 10⁻⁸ M, 10⁻⁹ M, 10⁻¹⁰ M, or less.

As used herein, the term "KD" refers to the equilibrium dissociation constant of a specific antibody-antigen interaction, and is used for describing the binding affinity between an antibody and an antigen. The smaller the equilibrium dissociation constant, the tighter the binding of the antibody to the antigen, and the higher the affinity between the antibody and the antigen. Typically, an antibody binds to its antigen with an affinity (KD) of less than about 10⁻⁵ M, for example, less than about 10⁻⁶ M, 10⁻⁷ M, 10⁻⁸ M, 10⁻⁹ M, 10⁻¹⁰ M, or less. Those skilled in the art can determine KD based on the common biological methods.

As used herein, the term "vector" generally refers to a nucleic acid molecule capable of being inserted into a suitable host for self-replication, which transfers the inserted nucleic acid molecule into and/or between host cells. The vector may include vectors primarily used for inserting DNA or RNA into cells, vectors primarily used for replicating DNA or RNA, and vectors primarily used for expressing DNA or RNA through transcription and/or translation. The term also encompasses vectors that have multiple functions as described above. The vector may be a polynucleotide capable of being transcribed and translated into a polypeptide when introduced into a suitable host cell. Typically, the vector can generate a desired expression product by culturing a suitable host cell including the vector.

As used herein, the term "composition" generally refers to a formulation in unit dosage form and can be prepared by any one of the methods well known in the pharmaceutical field. All such methods include the step of combining the active ingredient with a carrier that constitutes one or more auxiliary ingredients. Typically, the composition is prepared by uniformly and thoroughly combining an active compound with a liquid carrier, a finely divided solid carrier, or both.

As used herein, the term "pharmaceutically acceptable" refers to a substance that is suitable for use in humans and/or mammals without undue adverse side effects (such as toxicity, irritation, and allergic response), i.e., one that has a reasonable benefit/risk ratio.

As used herein, the term "pharmaceutically acceptable adjuvant" may include any solvent, solid excipient, diluent, or other liquid excipients, or the like suitable for specific target dosage forms. Except to the extent that any conventional adjuvant is incompatible with a compound of the present disclosure, for example, by causing undesirable biological effects or harmful interactions with any other components of the pharmaceutically acceptable composition, the use of such adjuvants is also contemplated within the scope of the present disclosure.

As used herein, the term "administering/administration" refers to introduction of a predetermined amount of a substance into a patient via a suitable route. The antibody or antigen-binding fragment, recombinant protein, bispecific antibody, conjugate, or pharmaceutical composition of the present disclosure can be administered via any common route, as long as it can reach intended tissues. Various routes of administration are contemplated, including intraperitoneal, intravenous, intramuscular, and subcutaneous injection, although the present disclosure is not limited to these exemplified administration routes. Preferably, the composition of the present disclosure is administered via intravenous or subcutaneous injection.

As used herein, the term "treat/treatment" refers to obtaining a desired pharmacological and/or physiological effect. Said effect may be prophylactic in terms of completely or partially prevention of a disease or its symptoms, and/or therapeutic in terms of partially or completely cure of a disease and/or adverse effects caused by the disease. The term "treat/treatment" as used herein encompasses treatment of diseases in mammals, particularly humans, including: (a) preventing the occurrence of a disease or condition in individuals who are susceptible to the disease but have not yet been diagnosed with it; (b) suppressing the disease, for example, arresting its progression; or (c) alleviating the disease, for example, reducing symptoms associated with the disease. The term "treat/treatment" as used herein encompasses any administration of a medicament or a compound to an individual for treating, curing, alleviating, ameliorating, relieving, or inhibiting the individual's disease, including but not limited to administering a medicament including a compound described herein to an individual in need thereof.

As used herein, the term "effective amount" or "effective dose" refers to an amount that may produce functions or activity to humans and/or animals and is acceptable to the humans and/or animals.

The amino acid sequences referred to herein are shown in Table 1.

The present disclosure provides an antibody targeting HLA-G or antigen-binding fragment thereof, an expression vector, a preparation method, a recombinant cell, an immunoconjugate, a composition, a pharmaceutical use, a medicament, use in the preparation a kit, a kit, a method for treating a disease, and use in treating a disease, which are described in detail below.

### Antibody or Antigen-Binding Fragment Thereof

In an aspect, the present disclosure provides an antibody or antigen-binding fragment thereof. The antibody or antigen-binding fragment thereof includes: a heavy chain complementarity determining region (HCDR) and a light chain complementarity determining region (LCDR). The aforementioned HCDR includes at least one amino acid sequence selected from SEQ ID NO: 1 to SEQ ID NO: 6, or an amino acid sequence having at least 80% homology thereto. The aforementioned LCDR includes at least one amino acid sequence selected from SEQ ID NO: 7 to SEQ ID NO: 12, or an amino acid sequence having at least 80% homology thereto. In embodiments of the present disclosure, the antibody or antigen-binding fragment thereof having the aforementioned HCDR and LCDR amino acid sequences can bind to both human and cynomolgus monkey HLA-G proteins with high affinity and specificity.

In embodiments of the present disclosure, the aforementioned HCDR includes HCDR1, HCDR2, and HCDR3. The aforementioned HCDR1 includes an amino acid sequence as set forth in SEQ ID NO: 1 or SEQ ID NO: 2, or an amino acid sequence having at least 80% homology to SEQ ID NO: 1 or SEQ ID NO: 2. The aforementioned HCDR2 includes an amino acid sequence as set forth in SEQ ID NO: 3 or SEQ ID NO: 4, or an amino acid sequence having at least 80% homology to SEQ ID NO: 3 or SEQ ID NO: 4. The aforementioned HCDR3 includes an amino acid sequence as set forth in SEQ ID NO: 5 or SEQ ID NO: 6, or an amino acid sequence having at least 80% homology to SEQ ID NO: 5 or SEQ ID NO: 6. The antibody or antigen-binding fragment thereof based on the aforementioned HCDR sequences has high binding affinity and specificity to HLA-G.

In some preferred embodiments of the present disclosure, the aforementioned HCDR includes: HCDR1 having an amino acid sequence as set forth in SEQ ID NO: 1 or SEQ ID NO: 2; HCDR2 having an amino acid sequence as set forth in SEQ ID NO: 3 or SEQ ID NO: 4; and HCDR3 having an amino acid sequence as set forth in SEQ ID NO: 5 or SEQ ID NO: 6. Experimental verification has shown that the antibody or antigen-binding fragment thereof having the amino acid sequences as set forth in SEQ ID NO: 1 to SEQ ID NO: 6 has high binding affinity and specificity to HLA-G.

In embodiments of the present disclosure, the aforementioned LCDR includes LCDR1, LCDR2, and LCDR3. The aforementioned LCDR1 includes an amino acid sequence as set forth in SEQ ID NO: 7 or SEQ ID NO: 8, or an amino acid sequence having at least 80% homology to SEQ ID NO: 7 or SEQ ID NO: 8. The aforementioned LCDR2 includes an amino acid sequence as set forth in SEQ ID NO: 9 or SEQ ID NO: 10, or an amino acid sequence having at least 80% homology to SEQ ID NO: 9 or SEQ ID NO: 10. The aforementioned LCDR3 includes an amino acid sequence as set forth in SEQ ID NO: 11 or SEQ ID NO: 12, or an amino acid sequence having at least 80% homology to SEQ ID NO: 11 or SEQ ID NO: 12. The antibody or antigen-binding fragment thereof based on the aforementioned LCDR sequences has high binding affinity and specificity to HLA-G.

In some preferred embodiments of the present disclosure, the aforementioned LCDR includes: LCDR1 having an amino acid sequence as set forth in SEQ ID NO: 7 or SEQ ID NO: 8; LCDR2 having an amino acid sequence as set forth in SEQ ID NO: 9 or SEQ ID NO: 10; and LCDR3 having an amino acid sequence as set forth in SEQ ID NO: 11 or SEQ ID NO: 12. Experimental verification has shown that the antibody or antigen-binding fragment thereof having the amino acid sequences as set forth in SEQ ID NO: 7 to SEQ ID NO: 12 has high binding affinity and specificity to HLA-G.

It should be noted that one or more amino acid residues in the HCDR and/or LCDR of the aforementioned antibody targeting HLA-G of the present disclosure may be substituted with other amino acid residues from the same side chain family, and the retained function of the modified antibody can be evaluated using the functional assays described herein. Preferably, the number of conservative modifications does not exceed one or two.

In embodiments of the present disclosure, the aforementioned antibody or antigen-binding fragment thereof further includes a heavy chain framework region.

In embodiments of the present disclosure, the aforementioned antibody or antigen-binding fragment thereof further includes a light chain framework region.

In embodiments of the present disclosure, at least a portion of the aforementioned heavy chain framework region and at least a portion of the aforementioned light chain framework region are independently from at least one of a rabbit antibody, a murine antibody, a sheep antibody, a goat antibody, a primate antibody, or a mutant thereof.

In some preferred embodiments of the present disclosure, a heavy chain variable region of the aforementioned antibody or antigen-binding fragment thereof includes an amino acid sequence as set forth in SEQ ID NO: 13 or SEQ ID NO: 14, or an amino acid sequence having at least 80% homology to SEQ ID NO: 13 or SEQ ID NO: 14. In some more preferred embodiments of the present disclosure, the amino acid sequence of the heavy chain variable region of the aforementioned antibody or antigen-binding fragment thereof is as set forth in SEQ ID NO: 13 or SEQ ID NO: 14.

In some preferred embodiments of the present disclosure, a light chain variable region of the aforementioned antibody or the antigen-binding fragment thereof includes an amino acid sequence as set forth in SEQ ID NO: 15 or SEQ ID NO: 16, or an amino acid sequence having at least 80% homology to SEQ ID NO: 15 or SEQ ID NO: 16. In some more preferred embodiments of the present disclosure, the amino acid sequence of the light chain variable region of the aforementioned antibody or antigen-binding fragment thereof is as set forth in SEQ ID NO: 15 or SEQ ID NO: 16.

The antibody or antigen-binding fragment thereof based on the aforementioned heavy chain variable region and light chain variable region sequences has high binding affinity and specificity to HLA-G.

In embodiments of the present disclosure, the aforementioned antibody or antigen-binding fragment thereof further includes a heavy chain constant region.

In embodiments of the present disclosure, the aforementioned antibody or antigen-binding fragment thereof further includes a light chain constant region.

In embodiments of the present disclosure, at least a portion of the aforementioned heavy chain constant region and at least a portion of the aforementioned light chain constant region are independently from at least one of a rabbit antibody, a murine antibody, a sheep antibody, a goat antibody, a primate antibody, or a mutant thereof.

In some preferred embodiments of the present disclosure, at least a portion of the aforementioned heavy chain constant region and at least a portion of the aforementioned light chain constant region are from a murine antibody or a human antibody.

In some preferred embodiments of the present disclosure, the aforementioned heavy chain constant region is selected from a human IgG1 constant region.

In some more preferred embodiments of the present disclosure, the aforementioned human IgG1 constant region has L234A/L235A mutation.

It should be noted that L234A indicates that leucine (Leu, L) at position 234 is mutated to alanine (Ala, A). Similarly, L235A indicates that leucine (Leu, L) at position 235 is mutated to alanine (Ala, A).

In some preferred embodiments of the present disclosure, the aforementioned light chain constant region is selected from a human Ig kappa constant region.

In embodiments of the present disclosure, a heavy chain of the aforementioned antibody or antigen-binding fragment thereof includes an amino acid sequence as set forth in SEQ ID NO: 17 or SEQ ID NO: 18, or an amino acid sequence having at least 80% homology to SEQ ID NO: 17 or SEQ ID NO: 18. In some preferred embodiments of the present disclosure, the amino acid sequence of the heavy chain of the aforementioned antibody or antigen-binding fragment thereof is as set forth in SEQ ID NO: 17 or SEQ ID NO: 18.

In embodiments of the present disclosure, a light chain of the aforementioned antibody or antigen-binding fragment thereof includes an amino acid sequence as set forth in SEQ ID NO: 19 or SEQ ID NO: 20, or an amino acid sequence having at least 80% homology to SEQ ID NO: 19 or SEQ ID NO: 20. In some preferred embodiments of the present disclosure, the amino acid sequence of the light chain of the aforementioned antibody or antigen-binding fragment thereof is as set forth in SEQ ID NO: 19 or SEQ ID NO: 20.

In embodiments of the present disclosure, the aforementioned antibody includes at least one selected from a polyclonal antibody and a monoclonal antibody; or the aforementioned antigen-binding fragment includes at least one selected from an F(ab')2 fragment, an Fab' fragment, a Fab fragment, an Fv fragment, an scFv fragment, an scFv-Fc fusion protein, an scFv-Fv fusion protein, and a minimal recognition unit. In some preferred embodiments of the present disclosure, the aforementioned antibody is a monoclonal antibody.

### Nucleic Acid Molecule

In another aspect, the present disclosure provides a nucleic acid molecule. The aforementioned nucleic acid molecule encodes the aforementioned antibody or antigen-binding fragment thereof. Based on the aforementioned nucleic acid molecule, *in vitro* large-scale preparation of the aforementioned antibody or antigen-binding fragment thereof can be achieved.

In embodiments of the present disclosure, the aforementioned nucleic acid molecule is DNA.

It should be noted that, with respect to the nucleic acid molecules referred to in the present specification and claims, those skilled in the art will appreciate that either strand or both strands of a complementary double-stranded nucleic acid are encompassed. For convenience, although only one strand is generally provided in the specification and claims, the complementary strand thereof is also disclosed. In addition, the nucleic acid sequences disclosed herein encompass both DNA and RNA forms. Disclosure of one form shall be understood to encompass disclosure of the other form as well.

### Expression Vector

In another aspect, the present disclosure provides an expression vector. The aforementioned expression vector carries the aforementioned nucleic acid molecule. Based on the aforementioned expression vector, *in vitro* large-scale preparation of the aforementioned antibody or antigen-binding fragment thereof can be achieved.

It should be noted that, when the above-mentioned nucleic acid molecule is linked to the vector, the nucleic acid molecule may be directly or indirectly linked to regulatory elements present in the vector, provided that such regulatory elements can control translation and expression of the aforementioned nucleic acid molecule. Of course, such regulatory elements may be directly from the vector, and they may also be exogenous, i.e., not from the vector itself. Of course, the aforementioned nucleic acid molecule is operatively linked to the regulatory elements.

According to an embodiment of the present disclosure, the aforementioned vector may refer to a cloning vector or an expression vector, and can be obtained by operatively linking the aforementioned nucleic acid to a commercially available vector, such as a plasmid or viral vector. The vector in the present disclosure is not particularly limited, and common plasmids such as pSeTag2, PEE14, and pMH3 can be used.

As used herein, the term "operably linked" refers to the linkage of a heterologous gene to a vector such that regulatory elements within the vector, including transcriptional control sequences and translational control sequences, are capable of exerting their intended regulatory functions on transcription and translation of the heterologous gene. Commonly used vectors may be, for example, viral vectors, plasmids, bacteriophages, etc. After the expression vector according to some specific embodiments of the present disclosure is introduced into suitable recipient cells, the expression of the aforementioned nucleic acid molecule can be effectively realized under the mediation of the regulatory system, thereby enabling the *in vitro* large-scale generation of proteins encoded by the nucleic acid molecule.

According to an embodiment of the present disclosure, the aforementioned vector is a eukaryotic vector or a prokaryotic vector.

According to an embodiment of the present disclosure, the aforementioned vector includes at least one selected from a plasmid vector, an adenoviral vector, a lentiviral vector, or an adeno-associated viral vector.

### Method for Preparing Antibody or Antigen-Binding Fragment Thereof

In another aspect, the present disclosure provides a method for preparing the aforementioned antibody or antigen-binding fragment thereof. The method includes: introducing the aforementioned expression vector into a cell; and culturing the aforementioned cell under conditions suitable for protein expression and secretion, to obtain the aforementioned antibody or antigen-binding fragment thereof. Through this method, *in vitro* large-scale preparation of the aforementioned antibody or antigen-binding fragment thereof can be achieved.

In embodiments of the present disclosure, the aforementioned cell is a eukaryotic cell.

In some specific examples of the present disclosure, the aforementioned eukaryotic cell is a mammalian cell. When the cell is the mammalian cell, expression efficiency of the aforementioned antibody or antigen-binding fragment is relatively high.

It should be noted that the aforementioned eukaryotic cell does not include an animal germ cell, fertilized egg, or embryonic stem cell.

### Recombinant Cell

In another aspect, the present disclosure provides a recombinant cell carrying the aforementioned antibody or antigen-binding fragment, the aforementioned nucleic acid molecule, or the aforementioned expression vector. In embodiments of the present disclosure, the aforementioned recombinant cell is obtained by transfecting or transforming the aforementioned expression vector, and can thus efficiently express the aforementioned antibody or antigen-binding fragment under suitable conditions.

In embodiments of the present disclosure, the aforementioned recombinant cell is a prokaryotic cell, a eukaryotic cell, or a bacteriophage. It should be noted that the aforementioned eukaryotic cell does not include an animal germ cell, fertilized egg, or embryonic stem cell.

In embodiments of the present disclosure, the aforementioned prokaryotic cell is *Escherichia coli*, *Bacillus subtilis*, *Streptomyces*, or *Proteus mirabilis*.

In embodiments of the present disclosure, the aforementioned eukaryotic cell is a fungus cell, an insect cell, a plant cell, or a mammalian cell.

In embodiments of the present disclosure, the aforementioned fungus is *Pichia pastoris*, *Saccharomyces cerevisiae*, *Schizosaccharomyces pombe*, or *Trichoderma*.

In embodiments of the present disclosure, the aforementioned insect cell is a *Spodoptera frugiperda* cell. In embodiments of the present disclosure, the aforementioned plant cell is tobacco plant cell. In embodiments of the present disclosure, the aforementioned mammalian cell is a BHK cell, a CHO cell, a COS cell, a myeloma cell, or a human embryonic kidney 293 (HEK293) cell, and does not include an animal germ cell, fertilized egg, or embryonic stem cell.

In embodiments of the present disclosure, the aforementioned recombinant cell is a mammalian cell. When the cell is the mammalian cell, the expression efficiency of the aforementioned antibody or antigen-binding fragment thereof is relatively high.

In embodiments of the present disclosure, the aforementioned recombinant cell is a BHK cell, a CHO cell, a COS cell, or an NSO cell.

It should be noted that "suitable conditions" as used herein refer to conditions suitable for expression of the antibody described herein. Those skilled in the art will readily appreciate that conditions suitable for the expression of the antibody include, but are not limited to, suitable transformation or transfection manners, suitable transformation or transfection conditions, healthy host cell state, suitable host cell density, suitable cell culture environment, and suitable cell culture time. The "suitable conditions" are not particularly limited, and one skilled in the art can optimize the optimal conditions for expression of the antibody according to the particular circumstances of the laboratory.

### Immunoconjugate

In another aspect, the present disclosure provides an immunoconjugate. The immunoconjugate includes: the aforementioned antibody or antigen-binding fragment thereof, the aforementioned nucleic acid molecule, the aforementioned expression vector, or the aforementioned recombinant cell. The aforementioned immunoconjugate can be used for treating an HLA-G related disease, such as a tumor.

In embodiments of the present disclosure, the aforementioned immunoconjugate may further include a therapeutic agent. The aforementioned therapeutic agent includes cytokines, immunomodulators, and small molecule drugs, etc.

### Composition

In another aspect, the present disclosure provides a composition. The composition includes: the aforementioned antibody or antigen-binding fragment thereof, the aforementioned nucleic acid molecule, the aforementioned expression vector, the aforementioned recombinant cell, or the aforementioned immunoconjugate. The aforementioned composition can be used for treating the HLA-G-related disease, such as a tumor.

It should be noted that the aforementioned composition includes combinations in which the components are separated in time and/or space, provided that they cooperate to achieve the purpose of the present disclosure. For example, the components contained in the aforementioned composition can be administered to subjects as a whole or separately. When the components contained in the aforementioned composition are administered separately to a subject, the individual components may be administered to the subject simultaneously or sequentially.

### Pharmaceutical Use

In yet another aspect, the present disclosure provides use of the aforementioned antibody or antigen-binding fragment thereof, the aforementioned nucleic acid molecule, the aforementioned expression vector, the aforementioned recombinant cell, the aforementioned immunoconjugate, or the aforementioned composition in the manufacture of a medicament for treating or preventing an HLA-G-mediated related disease. The medicament prepared based on the aforementioned antibody or antigen-binding fragment thereof can block the binding of HLA-G to LILRB1 or LILRB2, and significantly restore anti-tumor functions of NK cells and T cells.

In embodiments of the present disclosure, the aforementioned HLA-G-mediated related disease includes a tumor.

In embodiments of the present disclosure, the aforementioned tumor includes at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma.

### Medicament

In yet another aspect, the present disclosure provides a medicament. The medicament includes: the aforementioned antibody or antigen-binding fragment thereof, the aforementioned nucleic acid molecule, the aforementioned expression vector, the aforementioned recombinant cell, the aforementioned immunoconjugate, or the aforementioned composition. The medicament is for use in treating or preventing an HLA-G-mediated related disease. The aforementioned medicament can block the binding of HLA-G to LILRB1 or LILRB2, and significantly restore the anti-tumor functions of the NK cells and T cells.

In embodiments of the present disclosure, the aforementioned HLA-G-mediated related disease includes a tumor.

In embodiments of the present disclosure, the aforementioned tumor includes at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma.

In embodiments of the present disclosure, the medicament further includes a pharmaceutically acceptable adjuvant.

In embodiments of the present disclosure, the aforementioned adjuvant includes one or more pharmaceutically acceptable excipients, diluents, stabilizers, or carriers.

In embodiments of the present disclosure, the aforementioned pharmaceutical composition is in the form of an injection.

It should be noted that the aforementioned pharmaceutical composition includes combinations in which the components are separated in time and/or space, provided that they cooperate to achieve the purpose of the present disclosure. For example, the components contained in the aforementioned pharmaceutical composition can be administered to subjects as a whole or separately. When the components contained in the aforementioned pharmaceutical composition are administered separately to a subject, the individual components may be administered to the subject simultaneously or sequentially.

The medicament according to the present disclosure includes a safe and effective amount of active ingredients (the antibody or antigen-binding fragment) of the present disclosure and pharmaceutically acceptable adjuvants. These adjuvants include, but are not limited to: saline, buffer solution, glucose, water, glycerin, ethanol, and combinations thereof. Generally, pharmaceutical formulations should be compatible with the intended route of administration. Dosage forms of the medicament according to the present disclosure include injection, oral formulations (tablets, capsules, and oral liquid), transdermal formulations, and sustained-release formulations. For example, the medicament is prepared with normal saline or an aqueous solution containing glucose and other adjuvants by a conventional method. The medicament is preferably manufactured under sterile conditions.

An effective amount of the active ingredient according to the present disclosure may vary depending on factors such as the mode of administration and the severity of the disease to be treated. Selection of an appropriate effective amount may be determined by a person skilled in the art based on various factors, for example through clinical studies. Such factors include, but are not limited to, pharmacokinetic parameters of the active ingredient, such as bioavailability, metabolism, and half-life; severity of the disease to be treated in patient; patient body weight; patient immune status; and route of administration. For example, where treatment is urgently required, multiple divided doses may be administered per day, or the dosage may be proportionally adjusted.

The aforementioned pharmaceutically acceptable adjuvants according to the present disclosure include, but are not limited to, water, saline, liposomes, lipids, proteins, protein-antibody conjugates, peptides, cellulose, nanogels, or combinations thereof. Selection of a suitable carrier should be compatible with the route of administration and is well known to those skilled in the art.

### Use in Manufacture of Kit

In yet another aspect, the present disclosure provides use of the aforementioned antibody or antigen-binding fragment thereof in the manufacture of a kit for detecting HLA-G.

As previously described, the antibody or antigen-binding fragment thereof according to the embodiments of the present disclosure can specifically bind to HLA-G. Therefore, the aforementioned antibody or antigen-binding fragment thereof can be used for detecting HLA-G. Further, it can be used in kits for Western blotting, immunoprecipitation, and other detection methods that rely on the specific interaction between HLA-G and the antibody. Such kits may include one or more of the following: an antagonist, the antibody or antigen-binding fragment of the present disclosure, a reference material, a protein purification column, immunoglobulin affinity purification buffer, and a cell assay diluent. The antibody or antigen-binding fragment of the present disclosure can be used in different diagnostic tests, e.g., for detecting the presence of a wide variety of diseases or medicaments, toxins or other proteins, either in vitro or in vivo. For example, serum or blood from a subject may be analyzed to assess the presence of an HLA-G-mediated related disease.

### Kit

In yet another aspect, the present disclosure provides a kit including the aforementioned antibody or antigen-binding fragment thereof according to the first aspect. The aforementioned kit can be used for portable detection of HLA-G.

### Method for Treating Disease

In yet another aspect, the present disclosure provides a method for preventing and/or treating an HLA-G-mediated disease. According to an embodiment of the present disclosure, the aforementioned method includes: administering to a subject a pharmaceutically acceptable amount of the antibody or antigen-binding fragment thereof, the expression vector, the recombinant cell, the immunoconjugate, the composition, or the medicament. Based on the aforementioned method, the HLA-G-mediated related disease can be effectively treated.

It should be noted that the terms "subject", "individual", and "patient" may be used interchangeably herein and refer to a mammal being evaluated for treatment and/or being treated. In an embodiment, the mammal is a human. The terms "subject", "individual", and "patient" include, but are not limited to, individuals with tumors, individuals with autoimmune diseases, individuals with pathogen infection, and the like. The subject may be a human, but also includes other mammals, especially mammals that can be used as laboratory models for human diseases, such as mice and rats.

The effective amount of the aforementioned antibody or antigen-binding fragment thereof, nucleic acid molecule, expression vector, immunoconjugate, composition, or medicament according to the present disclosure may vary depending on factors such as the mode of administration and the severity of the disease to be treated. Selection of an appropriate effective amount may be determined by a person skilled in the art based on various factors, for example through clinical studies. Such factors include, but are not limited to, pharmacokinetic parameters of the active ingredient, such as bioavailability, metabolism, and half-life; severity of the disease to be treated in patient; patient body weight; patient immune status; and route of administration. For example, where treatment is urgently required, multiple divided doses may be administered per day, or the dosage may be proportionally adjusted.

In embodiments of the present disclosure, the aforementioned HLA-G-mediated related disease include a tumor.

In embodiments of the present disclosure, the aforementioned tumor includes at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma.

### Use in Treating Disease

In still yet another aspect, the present disclosure provides use of the aforementioned antibody or antigen-binding fragment thereof, the nucleic acid molecule, the expression vector, the immunoconjugate, the composition, or the medicament in the treatment or prevention of an HLA-G-mediated disease. In embodiments of the present disclosure, administering an effective dose of the antibody or antigen-binding fragment thereof, nucleic acid molecule, expression vector, immunoconjugate, composition, or medicament to the subject can effectively treat the HLA-G-mediated disease.

In embodiments of the present disclosure, the aforementioned HLA-G-mediated related disease includes a tumor.

In embodiments of the present disclosure, the aforementioned tumor includes at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma.

**[Table 1] HLA-G Antibody Sequences**

| SEQ ID NO: | Sequence | Description |
|---|---|---|
| 1 | TYLIH | Murine anti-HLA-G antibody HCDR1 |
| 2 | TYLIH | Humanized anti-HLA-G antibody HCDR1 |
| 3 | YINPYNDGSQYNENFKG | Murine anti-HLA-G antibody HCDR2 |
| 4 | YINPYNEGSQYNENFKG | Humanized anti-HLA-G antibody HCDR2 |
| 5 | GGNAMDY | Murine anti-HLA-G antibody HCDR3 |
| 6 | GGNAMDY | Humanized anti-HLA-G antibody HCDR3 |
| 7 | SASSSVSYIH | Murine anti-HLA-G antibody LCDR1 |
| 8 | SASSSVSYIH | Humanized anti-HLA-G antibody LCDR1 |
| 9 | STSNLAS | Murine anti-HLA-G antibody LCDR2 |
| 10 | STSNLAS | Humanized anti-HLA-G antibody LCDR2 |
| 11 | HQWRT | Murine anti-HLA-G antibody LCDR3 |
| 12 | HQWRT | Humanized anti-HLA-G antibody LCDR3 |
| 13 | EVQLQQSGPELVKPGASVKMSCKASGYTFTTYLIHWLKQKPGQ GLEWIGYINPYNDGSQYNENFKGKATLTSDKSSTTAYMELSSLTS EDSAVYYCSRGGNAMDYWGQGTSVTVSS | Murine anti-HLA-G antibody heavy chain variable region |
| 14 | QVQLVQSGAEVKKPGASVKVSCKASGYTFTTYLIHWLKQAPGQ GLEWIGYINPYNEGSQYNENFKGRATLTSDKSTSTVYMELSSLRS EDTAVYYCSRGGNAMDYWGQGTLVTVSS | Humanized anti-HLA-G antibody heavy chain variable region |
| 15 | QIVLTQSPAIMSASLGEEITLTCSASSSVSYIHWYQQKSGTSPKLLI YSTSNLASGVPSRFSGSGSGTFYSLTISSVEAEDAADYYCHQWRT FGGGTKLEIK | Murine anti-HLA-G antibody light chain variable region |
| 16 | DIQMTQSPSSLSASVGDRVTITCSASSSVSYIHWYQQKPGKAPKL LIYSTSNLASGVPSRFSGSGSGTDYTLTISSLQPEDFADYYCHQWR TFGGGTKLEIK | Humanized anti-HLA-G antibody light chain variable region |
| 17 | EVQLQQSGPELVKPGASVKMSCKASGYTFTTYLIHWLKQKPGQ GLEWIGYINPYNDGSQYNENFKGKATLTSDKSSTTAYMELSSLTS EDSAVYYCSRGGNAMDYWGQGTSVTVSSAKTTAPSVYPLAPVC GDTTGSSVTLGCLVKGYFPEPVTLTWNSGSLSSGVHTFPAVLQSD LYTLSSSVTVTSSTWPSQSITCNVAHPASSTKVDKKIEPRGPTIKPC PPCKCPAPNLLGGPSVFIFPPKIKDVLMISLSPIVTCVVVDVSEDDP DVQISWFVNNVEVHTAQTQTHREDYNSTLRVVSALPIQHQDWM SGKEFKCKVNNKDLPAPIERTISKPKGSVRAPQVYVLPPPEEEMT KKQVTLTCMVTDFMPEDIYVEWTNNGKTELNYKNTEPVLDSDG SYFMYSKLRVEKKNWVERNSYSCSVVHEGLHNHHTTKSFSRTP GK | Murine anti-HLA-G antibody heavy chain |
| 18 | QVQLVQSGAEVKKPGASVKVSCKASGYTFTTYLIHWLKQAPGQ GLEWIGYINPYNEGSQYNENFKGRATLTSDKSTSTVYMELSSLRS EDTAVYYCSRGGNAMDYWGQGTLVTVSSASTKGPSVFPLAPSSK STSGGTAALGCLVKDYFPEPVTVSWNSGALTSGVHTFPAVLQSSG LYSLSSVVTVPSSSLGTQTYICNVNHKPSNTKVDKKVEPKSCDKT HTCPPCPAPEAAGGPSVFLFPPKPKDTLMISRTPEVTCVVVDVSH EDPEVKFNWYVDGVEVHNAKTKPREEQYNSTYRVVSVLTVLHQ DWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLS PGK | Humanized anti-HLA-G antibody heavy chain |
| 19 | QIVLTQSPAIMSASLGEEITLTCSASSSVSYIHWYQQKSGTSPKLLI YSTSNLASGVPSRFSGSGSGTFYSLTISSVEAEDAADYYCHQWRT FGGGTKLEIKRADAAPTVSIFPPSSEQLTSGGASVVCFLNNFYPKD INVKWKIDGSERQNGVLNSWTDQDSKDSTYSMSSTLTLTKDEYE RHNSYTCEATHKTSTSPIVKSFNRNEC | Murine anti-HLA-G antibody light chain |
| 20 | DIQMTQSPSSLSASVGDRVTITCSASSSVSYIHWYQQKPGKAPKL LIYSTSNLASGVPSRFSGSGSGTDYTLTISSLQPEDFADYYCHQWR TFGGGTKLEIKRTVAAPSVFIFPPSDEQLKSGTASVVCLLNNFYPR EAKVQWKVDNALQSGNSQESVTEQDSKDSTYSLSSTLTLSKADY EKHKVYACEVTHQGLSSPVTKSFNRGEC | Humanized anti-HLA-G antibody light chain |
| 21 | ATGGTGGTCATGGCGCCCCGAACCCTCTTCCTGCTGCTCTCGG GGGCCCTGACCCTGACCGAGACCTGGGCGGGCTCCCACTCCA TGAGGTATTTCAGCGCCGCCGTGTCCCGGCCCGGCCGCGGGG AGCCCCGCTTCATCGCCATGGGCTACGTGGACGACACGCAGTT CGTGCGGTTCGACAGCGACTCGGCGTGTCCGAGGATGGAGCC GCGGGCGCCGTGGGTGGAGCAGGAGGGGCCGGAGTATTGGGA AGAGGAGACACGGAACACCAAGGCCCACGCACAGACTGACA GAATGAACCTGCAGACCCTGCGCGGCTACTACAACCAGAGCG AGGCCAGTTCTCACACCCTCCAGTGGATGATTGGCTGCGACCT GGGGTCCGACGGACGCCTCCTCCGCGGGTATGAACAGTATGCC TACGATGGCAAGGATTACCTCGCCCTGAACGAGGACCTGCGCT CCTGGACCGCAGCGGACACTGCGGCTCAGATCTCCAAGCGCA AGTGTGAGGCGGCCAATGTGGCTGAACAAAGGAGAGCCTACC TGGAGGGCACGTGCGTGGAGTGGCTCCACAGATACCTGGAGA ACGGGAAGGAGATGCTGCAGCGCGCGGACCCCCCCAAGACAC ACGTGACCCACCACCCTGTCTTTGACTATGAGGCCACCCTGAG GTGCTGGGCCCTGGGCTTCTACCCTGCGGAGATCATACTGACC TGGCAGCGGGATGGGGAGGACCAGACCCAGGACGTGGAGCTC GTGGAGACCAGGCCTGCAGGGGATGGAACCTTCCAGAAGTGG GCAGCTGTGGTGGTGCCTTCTGGAGAGGAGCAGAGATACACG TGCCATGTGCAGCATGAGGGGCTGCCGGAGCCCCTCATGCTGA GATGGAAGCAGTCTTCCCTGCCCACCATCCCCATCATGGGTATC GTTGCTGGCCTGGTTGTCCTTGCAGCTGTAGTCACTGGAGCTG CGGTCGCTGCTGTGCTGTGGAGAAAGAAGAGCTCAGATTGA | Human HLA-G gene sequence |

The solutions of the present disclosure will be further illustrated in conjunction with the examples. Those skilled in the art will understand that the examples described below are only used to illustrate the present disclosure and should not be considered as limiting the scope of the present disclosure. Unless otherwise indicated, the techniques or experimental conditions used in the examples are those described in the relevant literature in the art or in accordance with the manufacturers' instructions. Unless otherwise indicated, the reagents and instruments used are conventional commercially available products.

### Example 1: Preparation of Antibody

This example was conducted to obtain a murine monoclonal antibody against human HLA-G. Specifically, a His-tagged HLA-G protein (KACTUS Biosystems) was used as the as the immunogen and administered BALB/c mice together with complete Freund's adjuvant for three rounds of immunization. Blood was collected from the immunized mice, and serum was collected to detect immune responses. ELISA and flow cytometry were performed according to conventional procedures to screen sera and identify to obtain mice producing anti-human HLA-G immunoglobulins. Splenocytes isolated from the mouse exhibiting the highest anti-HLA-G immunoglobulin titer were fused with murine myeloma SP2/0 cells (ATCC No. CRL-1581). Hybridoma cells obtained after fusion were screened for antibody production to obtain the murine monoclonal antibody. The positive hybridoma clones were sequenced to obtain the sequences of the heavy chain variable region and light chain variable region of the murine monoclonal antibody. The amino acid sequences are as set forth in SEQ ID NO: 13 and SEQ ID NO: 15, respectively.

### Example 2: Evaluation of Binding Activity of Murine HLA-G Antibody

### Test Example 1: Evaluation of Binding Activity of Murine HLA-G Antibody to Human and Cynomolgus Monkey HLA-G Proteins

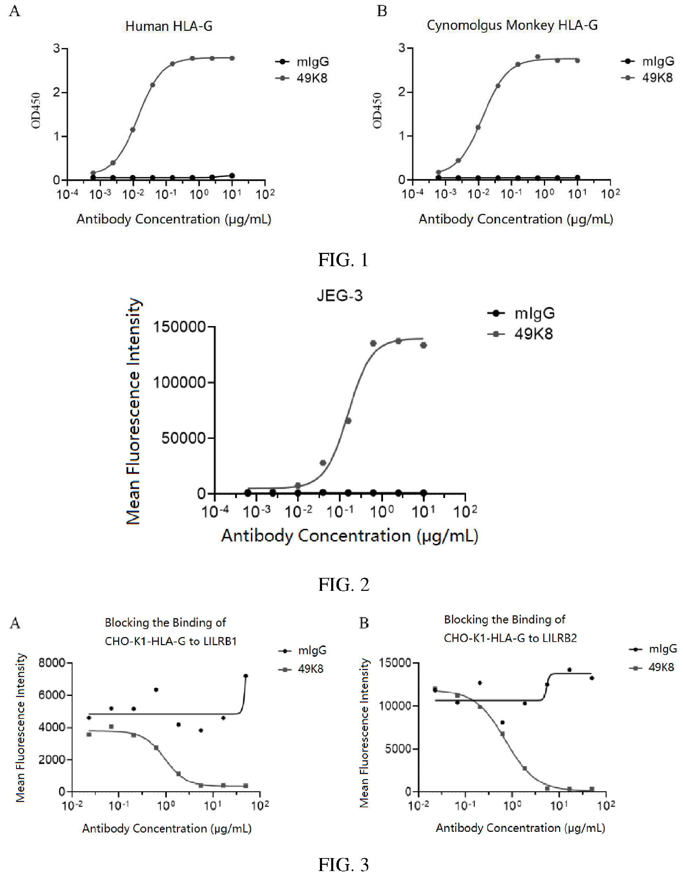

An ELISA assay was performed to detect the binding of the HLA-G antibody to human and cynomolgus monkey HLA-G proteins. The specific experimental procedures are as follows:
Human and cynomolgus monkey HLA-G proteins (KACTUS Biosystems) were diluted to 1 µg/mL with PBS buffer and added to a 96-well plate at a volume of 100 µL/well. The plate was placed at 4°C overnight. The PBS buffer in the 96-well plate was discarded. The plate was washed 3 times with PBST buffer (PBS containing 0.1% Tween 20, pH 7.2), and then 300 µL/well of 5% BSA was added. The plate was incubated at room temperature for 2 hours for blocking. The blocking solution was discarded, and 100 µL of test antibody diluted with 0.05% BSA was added. The plate was incubated at room temperature for 1 hour. The plate was washed 3 times with PBST, and then 100 µL of horseradish peroxidase (HRP)-labeled goat anti-mouse IgG Fab secondary antibody (Southern biotech) diluted with 0.5% BSA was added. The plate was incubated at room temperature for 1 hour. After the plate was washed 3 times with PBST, 80 µL/well of TMB was added, and the plate was incubated at room temperature for 10 minutes. The reaction was terminated by adding 80 µL/well of stop solution. Absorbance was measured at 450 nm using a microplate reader. The specific experimental results are shown in FIG. 1. The murine antibodyof the present disclosure (49K8) bound efficiently to both human and cynomolgus monkey HLA-G proteins.

### Test Example 2: Evaluation of Binding Activity of Murine HLA-G Antibody to JEG-3 Tumor Cell Line

Primary tumor cell line JEG-3 with high expression of HLA-G (ATCC No. HTB-36) was diluted to 1×10⁶ cells/mL in PBS and added to a 96-well plate at a volume of 90 µL/well. Subsequently, 10 µL of goat serum (Sangon Biotech, Shanghai) was added to each well and incubated at 4°C for 30 minutes for blocking. Serially diluted test antibodies were added and the cells were incubated at 4°C for 30 minutes. After incubation, the cells were washed twice with PBS. The cells were resuspended in 100 µL/well of PBS, followed by addition of 0.1 µL/well of APC-labeled goat anti-mouse IgG antibody (Biolegend), and incubated at 4°C in the dark for 30 minutes. The cells were washed twice with PBS. The cells were resuspended in 200 µL/well of PBS and analyzed by flow cytometry. As shown in FIG. 2, the murine antibody of the present disclosure (49K8) bound efficiently to the JEG-3 tumor cell line with high expression of HLA-G.

### Example 3: Evaluation of Blocking Activity of Murine HLA-G Antibody

### Test Example 1: Blocking of Binding of LILRB1 Protein or LILRB2 Protein to CHO-K1-HLA-G Cells by Murine HLA-G Antibody

The HLA-G antibody blocks the signaling pathway of HLA-G binding to its receptor LILRB1 or LILRB2 by binding to the extracellular domain of HLA-G. A flow cytometry assay was used to evaluate the blocking of the HLA-G antibody on the binding of HLA-G to its receptor. The specific experimental procedures are as follows:
HEK293T cells were seeded in a 6-well plate at 5×10⁵ cells/well and cultured overnight in DMEM medium without antibiotics. The medium was discarded before transfection, and 1 mL of fresh DMEM medium without antibiotics was added. Subsequently, pLVX-EF1a-human HLA-G-IRES-zsGreen (inserted with the nucleotide sequence encoding human HLA-G protein as set forth in SEQ ID NO: 21), pMD2G, and psPAX2 vectors (3 µg in total) were added at a ratio of 2:1:1 to 200 µL of serum-free DMEM medium. Then, 12 µg of polyethylenimine (PEI, Polysciences, Inc.) was added. After mixing well, the mixture was allowed to stand for 16 minutes, and then the entire liquid was added to the 6-well plate containing the above HEK293T cells. After the plate was incubated for 6 hours, the medium was discarded, and fresh complete DMEM medium was added for culture. After 48 hours of transfection, the cell culture supernatant was harvested and filtered through a 0.45 µm filter (Millipore) to obtain lentivirus supernatant. The obtained lentivirus supernatant was added entirely to a 6-well plate containing 1×10⁴ CHO-K1 cells, and polybrene (Sigma) was added at a final concentration of 4 µg/mL. The plate was incubated for 12 hours. Subsequently, the supernatant was completely discarded, and fresh complete DMEM medium was added. The resulting cells were designated CHO-K1-HLA-G cells.

The CHO-K1-HLA-G cells were diluted to 2×10⁶ cells/mL with PBS and added to a 96-well plate at a volume of 90 µL/well. Then, rat serum was added at 10 µL/well, and the cells were blocked at 4°C for 30 minutes. Serially diluted test antibodies and biotinylated human LILRB1 protein or LILRB2 protein (ACRO) at a final concentration of 2 µg/mL were added, and the cells were incubated at 4°C for 30 minutes. After incubation, the cells were washed twice with PBS. The cells were resuspended in 100 µL/well of PBS. Then, 0.1 µL/well of APC-labeled Streptavidin antibody (Biolegend) was added and incubated at 4°C in the dark for 30 minutes. The cells were washed twice with PBS, and the supernatant was discarded after centrifugation. The cells were resuspended in 200 µL/well of PBS and analyzed by flow cytometry. The specific experimental results are shown in FIG. 3. The murine antibody of the present disclosure (49K8) effectively blocked the binding of HLA-G to its receptor LILRB1 or LILRB2.

### Test Example 2: Blocking of Binding of HLA-G Protein to LILRB1 Protein or LILRB2 Protein by Murine HLA-G Antibody

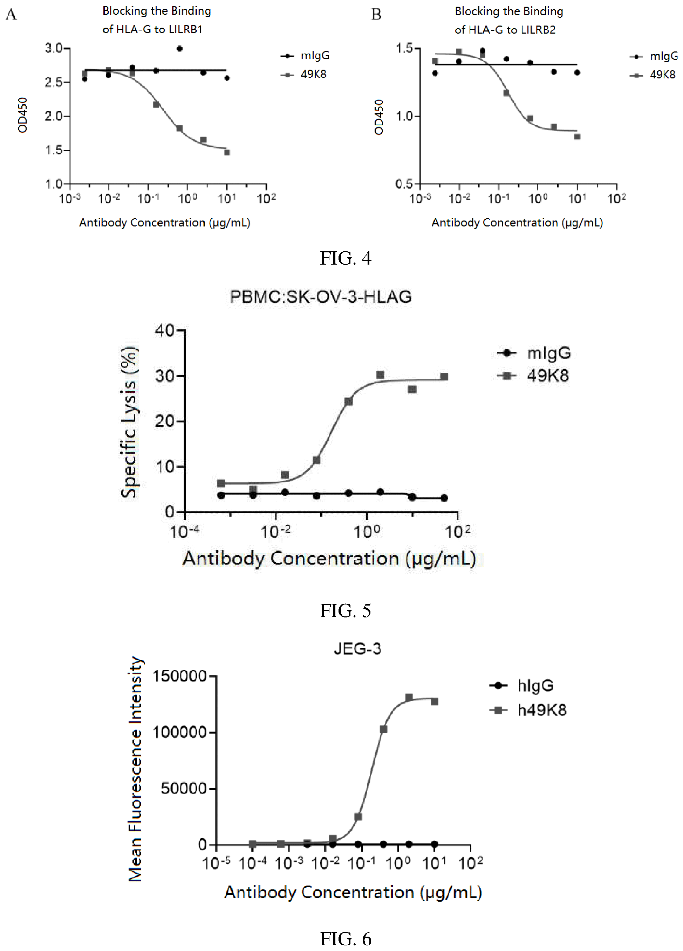

An ELISA assay was performed to detect the blocking of the binding of HLA-G protein to LILRB1 protein or LILRB2 protein by the HLA-G antibody. The specific experimental procedures are as follows:
Human HLA-G protein (KACTUS Biosystems) was diluted to 5 µg/mL with PBS buffer and added to a 96-well plate at a volume of 100 µL/well. The plate was placed at 4°C overnight. The PBS buffer in the 96-well plate was discarded. The plate was washed 3 times with PBST buffer (PBS containing 0.1% Tween 20, pH 7.2), and then 300 µL/well of 5% BSA was added. The plate was incubated at room temperature for 2 hours for blocking. The blocking solution was discarded, and biotin-labeled LILRB1 protein or LILRB2 protein (ACRO) diluted to 2 µg/mL in 0.05% BSA was added at 50 µL/well together with 50 µL of test antibody diluted in 0.05% BSA. The plate was incubated at room temperature for 1 hour. The plate was washed 3 times with PBST, and then 100 µL of HRP-labeled Streptavidin secondary antibody (Southern biotech) diluted in 0.5% BSA was added. The plate was incubated at room temperature for 1 hour. After the plate was washed 3 times with PBST, 80 µL/well of TMB was added, and the plate was incubated at room temperature for 10 minutes. The reaction was terminated with 80 µL/well stop solution. Absorbance at 450 nm was measured using a microplate reader. The specific experimental results are shown in FIG. 4. The murine antibody of the present disclosure (49K8) effectively blocked the binding of HLA-G to its receptor LILRB1 or LILRB2.

### Example 4: Evaluation of Activity of Murine Antibody in Promoting PBMC-mediated Killing

A flow cytometry assay was used to detect the activity of the HLA-G antibody in promoting PBMC-mediated killing.

HEK293T cells were seeded in a 6-well plate at 5×10⁵ cells/well and cultured overnight in DMEM medium without antibiotics. The medium was discarded before transfection, and 1 mL of fresh DMEM medium without antibiotics was added. Subsequently, pLVX-EF1a-human HLA-G-IRES-zsGreen (inserted with the nucleotide sequence encoding human HLA-G protein as set forth in SEQ ID NO: 21), pMD2G, and psPAX2 vectors (3 µg in total) were added at a ratio of 2:1:1 to 200 µL of serum-free DMEM medium. Then, 12 µg of polyethylenimine (PEI, Polysciences, Inc.) was added. After mixing well, the mixture was allowed to stand for 16 minutes, and then the entire liquid was added to the 6-well plate containing the above HEK293T cells. After the plate was incubated for 6 hours, the medium was discarded, and fresh complete DMEM medium was added for culture. After 48 hours of transfection, the cell culture supernatant was harvested and filtered through a 0.45 µm filter (Millipore) to obtain lentivirus supernatant. The obtained lentivirus supernatants were separately added entirely to a 6-well plate containing 1×10⁴ SK-OV-3 cells (ATCC No. HTB-77) or K562 cells (ATCC No. CCL-243), and polybrene (Sigma) was added at a final concentration of 4 µg/mL. The plate was incubated for 12 hours. Subsequently, the supernatant was completely discarded, and fresh complete DMEM medium was added. The resulting cells were designated SK-OV-3-HLA-G or K562-HLA-G cells.

The SK-OV-3-HLA-G cells were collected, resuspended in 1640 medium, counted, and adjusted to a cell density of approximately 1.5×10⁶ cells/mL. Subsequently, 1 mL of cell suspension was transferred into a 1.5 mL EP tube, and 1 µL of Cell Trace Violet (Invitrogen) was added. After mixing, the cells were labeled at 37°C for 20 minutes. The labeled cells were washed 3 times with 1640 medium, and adjusted to a cell density of 1×10⁵ cells/mL. PBMCs (MileCell Bio) were collected by centrifugation at 300 g, resuspended in 1640 medium, counted, and adjusted to a cell density of 5×10⁶ cells/mL. In a 96-well round-bottom plate, 100 µL/well of the diluted SK-OV-3-HLA-G cells and 100 µL/well of PBMCs were added, along with serially diluted test antibodies. The plate was centrifuged at 250 g for 4 minutes at room temperature, and then incubated in an incubator with 5% CO₂ at 37°C for 4 hours to 6 hours. After incubation, 1 µL of 7AAD (BD Biosciences) was added to each well and mixed thoroughly. Flow cytometric analysis was then performed. The specific experimental results are shown in FIG. 5. The murine antibody of the present disclosure (49K8) effectively promoted PBMC-mediated killing of SK-OV-3 cell line overexpressing HLA-G in a dose-dependent manner.

### Example 5: Humanization of Antibody

Based on the heavy chain variable region sequence (as set forth in SEQ ID NO: 13) and the light chain variable region sequence (as set forth in SEQ ID NO: 15) of the HLA-G antibody obtained in Example 1, humanization templates showing the highest compatibility with the non-CDR regions were identified using MOE software. The CDRs of the murine antibody were grafted onto the selected humanized template to replace the CDRs of the human template, thereby obtaining the humanized antibody. Then, based on the three-dimensional structure of the murine antibody, back-mutations were introduced into buried residues, residues directly interacting with the CDRs, and FR residues that significantly influence the conformation of VL and VH domains, thereby obtaining the humanized antibody. In addition, some chemical modifications, known as post-translational modifications (PTMs), are introduced after cellular translation of proteins. For antibodies, certain PTMs present in the CDRs are likely to substantially affect antigen binding and may also alter the physicochemical properties of antibodies, thereby increasing difficulty and risk in antibody development. In order to avoid these potential PTM risks, amino acid mutations were introduced at these PTM sites during the humanization process to eliminate the formation of specific PTMs. The final heavy chain variable region sequence of the obtained humanized HLA-G antibody is as set forth in SEQ ID NO: 14, and the final light chain variable region sequenc is as set forth in SEQ ID NO: 16. The heavy chain constant region of the aforementioned humanized antibody (h49K8) is a human IgG1 constant region with L234A/L235A mutations introduced, and its light chain constant region is a human Ig kappa constant region.

### Example 6: Evaluation of Binding Activity of Humanized Antibody

A flow cytometry assay was used to detect the ability of the humanized HLA-G antibody to bind to JEG-3 cell line.

JEG-3 cells were diluted to 1×10⁶ cells/mL with PBS and added to a 96-well plate at a volume of 90 µL/well. Subsequently, 10 µL of goat serum (Sangon Biotech, Shanghai) was added to each well and incubated at 4°C for 30 minutes for blocking. Serially diluted test antibodies were added and the cells were incubated at 4°C for 30 minutes. After incubation, the cells were washed twice with PBS. The cells were resuspended in 100 µL/well of PBS, followed by addition of 0.1 µL/well of AF647-labeled goat anti-human IgG Fc-specific antibody (Jackson ImmunoResearch), and incubated at 4°C in the dark for 30 minutes. The cells were washed twice with PBS. The cells were resuspended in 200 µL/well of PBS and analyzed by flow cytometry. As shown in FIG. 6, the humanized antibody of the present disclosure (h49K8) bound efficiently to the JEG-3 tumor cell line with high expression of HLA-G.

### Example 7: Evaluation of Blocking Activity of Humanized Antibody

### Test Example 1: Blocking of Binding of LILRB1 Protein or LILRB2 Protein to CHO-K1-HLA-G Cells by Humanized HLA-G Antibody

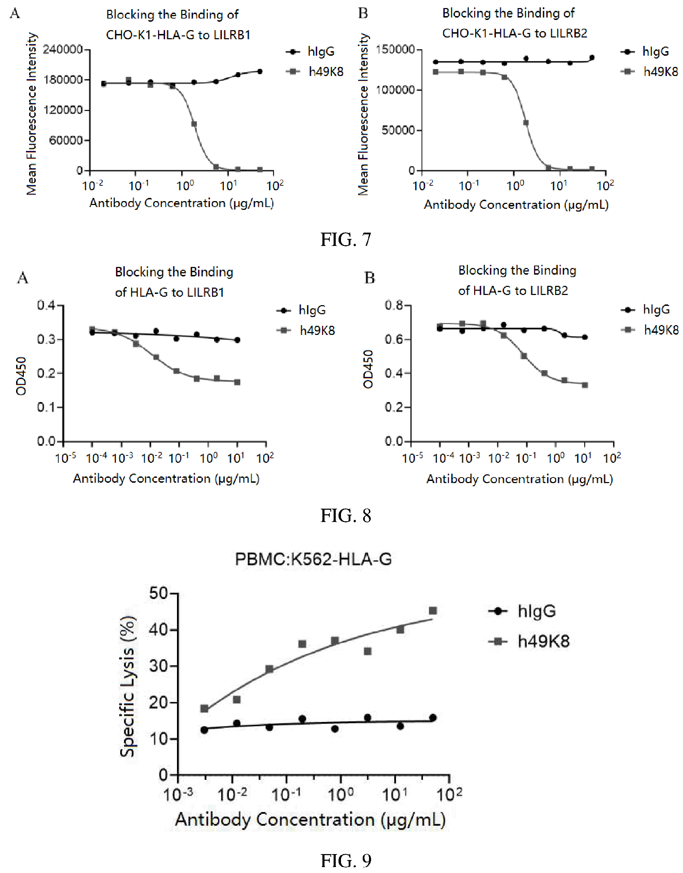

A flow cytometry assay was used to detect the blocking of the humanized HLA-G antibody on the binding of LILRB1 protein or LILRB2 protein to CHO-K1-HLA-G cells. The specific experimental procedures are as follows:
The CHO-K1-HLA-G cells were diluted to 2×10⁶ cells/mL with PBS and added to a 96-well plate at a volume of 90 µL/well. Then, rat serum was added at 10 µL/well, and the cells were blocked at 4°C for 30 minutes. Serially diluted test antibodies and biotinylated human LILRB1 protein or LILRB2 protein (ACRO) at a final concentration of 2 µg/mL were added, and the cells were incubated at 4°C for 30 minutes. After incubation, the cells were washed twice with PBS. The cells were resuspended in 100 µL/well of PBS. Then, 0.1 µL/well of APC-labeled Streptavidin antibody (Biolegend) was added and incubated at 4°C in the dark for 30 minutes. The cells were washed twice with PBS, and the supernatant was discarded after centrifugation. The cells were resuspended in 200 µL/well of PBS and analyzed by flow cytometry. The specific experimental results are shown in FIG. 7. The humanized antibody of the present disclosure (h49K8) effectively blocked the binding of HLA-G to its receptor LILRB1 or LILRB2.

### Test Example 2: Blocking of Binding of HLA-G Protein to LILRB1 Protein or LILRB2 Protein by Humanized HLA-G Antibody

An ELISA assay was used to detect the blocking of the binding of HLA-G protein to LILRB1 protein or LILRB2 protein by the HLA-G antibody. The specific experimental procedures are as follows:
Human HLA-G protein (KACTUS Biosystems) was diluted to 5 µg/mL with PBS buffer and added to a 96-well plate at a volume of 100 µL/well. The plate was placed at 4°C overnight. The PBS buffer in the 96-well plate was discarded. The plate was washed 3 times with PBST buffer (PBS containing 0.1% Tween 20, pH 7.2), and then 300 µL/well of 5% BSA was added. The plate was incubated at room temperature for 2 hours for blocking. The blocking solution was discarded, and biotin-labeled LILRB1 protein or LILRB2 protein (ACRO) diluted to 2 µg/mL in 0.05% BSA was added at 50 µL/well together with 50 µL of test antibody diluted in 0.05% BSA. The plate was incubated at room temperature for 1 hour. The plate was washed 3 times with PBST, and then 100 µL of HRP-labeled Streptavidin secondary antibody (Southern biotech) diluted in 0.5% BSA was added. The plate was incubated at room temperature for 1 hour. After the plate was washed 3 times with PBST, 80 µL/well of TMB was added, and the plate was incubated at room temperature for 10 minutes. The reaction was terminated with 80 µL/well stop solution. Absorbance at 450 nm was measured using a microplate reader. The specific experimental results are shown in FIG. 8. The humanized antibody of the present disclosure (h49K8) effectively blocked the binding of HLA-G to its receptor LILRB1 or LILRB2.

### Example 8: Evaluation of Activity of Humanized Antibody in Promoting PBMC-mediated Killing

A flow cytometry assay was used to detect the activity of the humanized HLA-G antibody in promoting PBMC-mediated killing.

K562-HLA-G cells were collected, resuspended in 1640 medium, counted, and adjusted to a cell density of approximately 1.5×10⁶ cells/mL. Subsequently, 1 mL of cell suspension was transferred into a 1.5 mL EP tube, and 1 µL of Cell Trace Violet (Invitrogen) was added. After mixing, the cells were labeled at 37°C for 20 minutes. The labeled cells were washed 3 times with 1640 medium, and adjusted to a cell density of 1×10⁵ cells/mL. PBMCs were collected by centrifugation at 300 g, resuspended in 1640 medium, counted, and adjusted to a cell density of 1×10⁶ cells/mL. In a 96-well round-bottom plate, 100 µL/well of the diluted K562-HLA-G cells and 100 µL/well of PBMCs were added, along with serially diluted test antibodies. The plate was centrifuged at 250 g for 4 minutes at room temperature, and then incubated in an incubator with 5% CO₂ at 37°C for 4 hours to 6 hours. After incubation, 1 µL of 7AAD (BD Biosciences) was added to each well and mixed thoroughly. Flow cytometric analysis was then performed. The specific experimental results are shown in FIG. 9. The humanized antibody of the present disclosure (49K8) effectively promoted PBMC-mediated killing of K562 cell line overexpressing HLA-G in a dose-dependent manner.

### Example 9: Affinity Determination of Humanized Antibody

Surface plasmon resonance (SPR) technology was used to determine the affinity of the humanized HLA-G antibody.

The specific procedures are as follows: A Biacore^{™} 1K instrument (Cytiva) was initialized, and a Protein A chip (Cytiva) was installed and equilibrated in HBS-EP+buffer. The test antibody at a concentration of 1 µg/mL was then passed over the chip surface at a flow rate of 30 µL/min to capture the test antibody. Human HLA-G protein (KACTUS Biosystems) or cynomolgus monkey HLA-G protein (KACTUS Biosystems) at different concentrations was passed over the chip surface at a flow rate of 30 µL/min, with an association time set to 90 seconds, followed by dissociation for 600 seconds. Kinetic analysis was performed using a 1:1 binding model. The affinity values of the humanized HLA-G antibody binding to human and cynomolgus monkey HLA-G proteins are shown in Table 2, indicating that the humanized HLA-G antibody exhibited significant binding activity to both human and cynomolgus monkey HLA-G proteins.

**[Table 2] Affinity Values of the Humanized HLA-G Antibody**

| Antibody | Antigen | Ka (1/Ms) | Kd (1/s) | KD (M) |
|---|---|---|---|---|
| h49K8 | Human HLA-G | 7.40E+05 | 4.05E-04 | 5.48E-10 |
| h49K8 | Monkey HLA-G | 9.28E+04 | 5.96E-03 | 6.42E-08 |

The foregoing results demonstrate that the monoclonal antibody prepared in the present disclosure has high specificity and affinity, effectively binds to both human and cynomolgus monkey HLA-G proteins, blocks the binding of HLA-G to LILRB1 or LILRB2 proteins, and promotes the killing ability of immune cells (e.g., PBMCs) against tumor cells.

The preferred embodiments of the present disclosure are described in detail above. However, the present disclosure is not limited thereto. Without departing from the scope of the technical concept of the present disclosure, various simple modifications can be made to the technical solutions of the present disclosure, including the combination of various technical features in any other suitable manner. These simple modifications and combinations should also be regarded as the disclosed content of the present disclosure and fall within the protection scope of the present disclosure.

In the description of the present specification, the descriptions of the reference terms such as "an embodiment", "some embodiments", "an example", "a specific example", and "some examples" mean that the specific features, structures, materials or characteristics described in combination with the embodiments or examples are included in at least one embodiment or example of the present disclosure. In the present specification, the schematic expressions of the above terms do not necessarily refer to the same embodiment or example. Moreover, the specific features, structures, materials or characteristics described herein may be combined in any one or more embodiments or examples in an appropriate mode. In addition, in absence of mutual contradiction, different embodiments or examples and features of the different embodiments or examples described in the present specification may be combined and integrated by those skilled in the art.

Although the embodiments of the present disclosure have been illustrated and described above, it can be understood that the above embodiments are illustrative and should not be construed as limiting the present disclosure. Those skilled in the art can make changes, modifications, substitutions, and variations to the above embodiments within the scope of the present disclosure.

## Claims

1. An antibody or antigen-binding fragment thereof, comprising:
a heavy chain complementarity determining region (HCDR), wherein the HCDR comprises at least one amino acid sequence selected from SEQ ID NO: 1 to SEQ ID NO: 6, or an amino acid sequence having at least 80% homology thereto; and
a light chain complementarity determining region (LCDR), wherein the LCDR comprises at least one amino acid sequence selected from SEQ ID NO: 7 to SEQ ID NO: 12, or an amino acid sequence having at least 80% homology thereto.

2. The antibody or antigen-binding fragment thereof according to claim 1, wherein the HCDR comprises HCDR1, HCDR2, and HCDR3, wherein:
the HCDR1 comprises an amino acid sequence as set forth in SEQ ID NO: 1 or SEQ ID NO: 2, or an amino acid sequence having at least 80% homology to SEQ ID NO: 1 or SEQ ID NO: 2;
the HCDR2 comprises an amino acid sequence as set forth in SEQ ID NO: 3 or SEQ ID NO: 4, or an amino acid sequence having at least 80% homology to SEQ ID NO: 3 or SEQ ID NO: 4; and
the HCDR3 comprises an amino acid sequence as set forth in SEQ ID NO: 5 or SEQ ID NO: 6, or an amino acid sequence having at least 80% homology to SEQ ID NO: 5 or SEQ ID NO: 6;
preferably, the HCDR comprises HCDR1 having an amino acid sequence as set forth in SEQ ID NO: 1 or SEQ ID NO: 2;
preferably, the HCDR comprises HCDR2 having an amino acid sequence as set forth in SEQ ID NO: 3 or SEQ ID NO: 4; and
preferably, the HCDR comprises HCDR3 having an amino acid sequence as set forth in SEQ ID NO: 5 or SEQ ID NO: 6.

3. The antibody or antigen-binding fragment thereof according to claim 1, wherein the LCDR comprises LCDR1, LCDR2, and LCDR3, wherein:
the LCDR1 comprises an amino acid sequence as set forth in SEQ ID NO: 7 or SEQ ID NO: 8, or an amino acid sequence having at least 80% homology to SEQ ID NO: 7 or SEQ ID NO: 8,
the LCDR2 comprises an amino acid sequence as set forth in SEQ ID NO: 9 or SEQ ID NO: 10, or an amino acid sequence having at least 80% homology to SEQ ID NO: 9 or SEQ ID NO: 10, and
the LCDR3 comprises an amino acid sequence as set forth in SEQ ID NO: 11 or SEQ ID NO: 12, or an amino acid sequence having at least 80% homology to SEQ ID NO: 11 or SEQ ID NO: 12;
preferably, the LCDR comprises LCDR1 having an amino acid sequence as set forth in SEQ ID NO: 7 or SEQ ID NO: 8;
preferably, the LCDR comprises LCDR2 having an amino acid sequence as set forth in SEQ ID NO: 9 or SEQ ID NO: 10;
preferably, the LCDR comprises LCDR3 having an amino acid sequence as set forth in SEQ ID NO: 11 or SEQ ID NO: 12.

4. The antibody or antigen-binding fragment thereof according to claim 2 or 3, further comprising:
a heavy chain framework region; and
optionally, a light chain framework region, wherein:
optionally, at least a portion of the heavy chain framework region and at least a portion of the light chain framework region are independently from at least one of a rabbit antibody, a murine antibody, a sheep antibody, a goat antibody, a primate antibody, or a mutant thereof;
preferably, a heavy chain variable region of the antibody or antigen-binding fragment thereof comprises an amino acid sequence as set forth in SEQ ID NO: 13 or SEQ ID NO: 14, or an amino acid sequence having at least 80% homology to SEQ ID NO: 13 or SEQ ID NO: 14; and preferably, the amino acid sequence of the heavy chain variable region of the antibody or antigen-binding fragment thereof is as set forth in SEQ ID NO: 13 or SEQ ID NO: 14; and
preferably, a light chain variable region of the antibody or the antigen-binding fragment thereof comprises an amino acid sequence as set forth in SEQ ID NO: 15 or SEQ ID NO: 16, or an amino acid sequence having at least 80% homology to SEQ ID NO: 15 or SEQ ID NO: 16; and preferably, the amino acid sequence of the light chain variable region of the antibody or antigen-binding fragment thereof is as set forth in SEQ ID NO: 15 or SEQ ID NO: 16.

5. The antibody or antigen-binding fragment thereof according to claim 4, further comprising a heavy chain constant region; and
optionally, a light chain constant region, wherein:
optionally, at least a portion of the heavy chain constant region and at least a portion of the light chain constant region are independently from at least one of a rabbit antibody, a murine antibody, a sheep antibody, a goat antibody, a primate antibody, or a mutant thereof, and preferably, at least a portion of the heavy chain constant region and at least a portion of the light chain constant region are from a murine antibody or a human antibody;
preferably, the heavy chain constant region is selected from a human IgG1 constant region, and more preferably, the human IgG1 constant region has L234A/L235A mutation;
preferably, the light chain constant region is selected from a human Ig kappa constant region;
preferably, a heavy chain of the antibody or antigen-binding fragment thereof comprises an amino acid sequence as set forth in SEQ ID NO: 17 or SEQ ID NO: 18, or an amino acid sequence having at least 80% homology to SEQ ID NO: 17 or SEQ ID NO: 18; and preferably, the amino acid sequence of the heavy chain of the antibody or antigen-binding fragment thereof is as set forth in SEQ ID NO: 17 or SEQ ID NO: 18; and
preferably, a light chain of the antibody or antigen-binding fragment thereof comprises an amino acid sequence as set forth in SEQ ID NO: 19 or SEQ ID NO: 20, or an amino acid sequence having at least 80% homology to SEQ ID NO: 19 or SEQ ID NO: 20; and preferably, the amino acid sequence of the light chain of the antibody or antigen-binding fragment thereof is as set forth in SEQ ID NO: 19 or SEQ ID NO: 20.

6. The antibody or antigen-binding fragment thereof according to claim 1, wherein:
the antibody comprises at least one selected from a polyclonal antibody and a monoclonal antibody; or
the antigen-binding fragment comprises at least one selected from an F(ab')2 fragment, an Fab' fragment, a Fab fragment, an Fv fragment, an scFv fragment, an scFv-Fc fusion protein, an scFv-Fv fusion protein, and a minimal recognition unit; and
preferably, the antibody is a monoclonal antibody.

7. A nucleic acid molecule, encoding the antibody or antigen-binding fragment thereof according to any one of claims 1 to 6,
optionally, the nucleic acid molecule is DNA.

8. An expression vector, carrying the nucleic acid molecule according to claim 7.

9. A method for preparing the antibody or antigen-binding fragment thereof according to any one of claims 1 to 6, the method comprising:
introducing the expression vector according to claim 8 into cells; and
culturing the cells under conditions suitable for protein expression and secretion, to obtain the antibody or antigen-binding fragment thereof, wherein the cells are optionally eukaryotic cells.

10. A recombinant cell, carrying the nucleic acid molecule according to claim 7 or the expression vector according to claim 8.

11. An immunoconjugate, comprising:
the antibody or antigen-binding fragment thereof according to any one of claims 1 to 6, the nucleic acid molecule according to claim 7, the expression vector according to claim 8, or the recombinant cell according to claim 10; and
optionally, a therapeutic agent.

12. A composition, comprising:
the antibody or antigen-binding fragment thereof according to any one of claims 1 to 6, the nucleic acid molecule according to claim 7, the expression vector according to claim 8, the recombinant cell according to claim 10, or the immunoconjugate according to claim 11.

13. Use of the antibody or antigen-binding fragment thereof according to any one of claims 1 to 6, the nucleic acid molecule according to claim 7, the expression vector according to claim 8, the recombinant cell according to claim 10, the immunoconjugate according to claim 11, or the composition according to claim 12 in the manufacture of a medicament for treating or preventing an HLA-G-mediated related disease.

14. The use according to claim 13, wherein:
the HLA-G-mediated related disease comprises a tumor; and
optionally, the tumor comprises at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma.

15. A medicament, comprising:
the antibody or antigen-binding fragment thereof according to any one of claims 1 to 6, the nucleic acid molecule according to claim 7, the expression vector according to claim 8, the recombinant cell according to claim 10, the immunoconjugate according to claim 11, or the composition according to claim 12, wherein the medicament is for use in the treatment or prevention of an HLA-G-mediated related disease.

16. The medicament according to claim 15, wherein:
the HLA-G-mediated related disease comprises a tumor;
optionally, the tumor comprises at least one of breast cancer, colorectal cancer, cervical cancer, endometrial cancer, esophageal cancer, gastric cancer, hepatocellular carcinoma, lung cancer, nasopharyngeal carcinoma, ovarian cancer, leukemia, or renal cell carcinoma;
optionally, the medicament further comprises a pharmaceutically acceptable adjuvant;
optionally, the adjuvant comprises one or more pharmaceutically acceptable excipients, diluents, stabilizers, or carriers; and
optionally, the medicament is in the form of an injection.

17. Use of the antibody or antigen-binding fragment thereof according to any one of claims 1 to 6 in the manufacture of a kit for detecting HLA-G.

18. A kit, comprising:
the antibody or the antigen-binding fragment thereof according to any one of claims 1 to 6.

19. The kit according to claim 18, wherein the kit is for use in detecting HLA-G.

20. A method for preventing and/or treating an HLA-G-mediated disease, the method comprising:
administering to a subject a pharmaceutically acceptable amount of the antibody or antigen-binding fragment thereof according to any one of claims 1 to 6, the expression vector according to claim 8, the recombinant cell according to claim 10, the immunoconjugate according to claim 11, the composition according to claim 12, or the medicament according to claim 15.
